# EUROPÄISCHE PATENTSCHRIFT

(11) **EP 4 294 271 B1**
(45) Veröffentlichungstag und Bekanntmachung des Hinweises auf die Patenterteilung: **31.12.2025**
(21) Anmeldenummer: 23717406.5
(22) Anmeldetag: 12.04.2023
(51) Int. Cl.: A61B 5/268, A61B 5/27

(54) **ELEKTRODE FÜR EIN BEKLEIDUNGSSTÜCK, EINEN GURT ODER EINE BANDAGE**
ELECTRODE FOR AN ITEM OF CLOTHING, A STRAP OR A BANDAGE
ÉLECTRODE DESTINÉ À UN VÊTEMENT, UNE SANGLE OU UN BANDAGE

(30) Priorität: 29.04.2022 DE 102022110523
(43) Veröffentlichungstag der Anmeldung: 27.12.2023
(73) Patentinhaber: NTT New Textile Technologies GmbH, 72336 Balingen (DE)
(72) Erfinder: Baure, Hans, 73226 Balingen (DE)
(74) Vertreter: Müller, Gottfried
(86) Internationale Anmeldenummer: PCT/EP2023/025172
(87) Internationale Veröffentlichungsnummer: WO 2023/208413

(56) Entgegenhaltungen:
- EP-A2- 2 036 496
- WO-A1-2020/196975
- WO-A1-2021/134131
- WO-A2-2022/061250
- DE-B4- 112004 001 921
- DE-U1- 202018 100 556
- US-A1- 2021 007 223

## Beschreibung

Die Erfindung bezieht sich auf eine Elektrode für ein Bekleidungsstück, einen Gurt oder eine Bandage. Die Erfindung bezieht sich des Weiteren auf eine Stofflage-Elektrode-Kombination mit einer Elektrode und auf ein Bekleidungsstück, einen Gurt oder eine Bandage mit einer Stofflage-Elektrode-Kombination, wobei die Elektrode mit einer Stofflage des Bekleidungsstücks, des Gurts oder der Bandage verbunden ist.

Bekannt sind Elektroden in Form von der Dehnungssensoren, die auf eine Stofflage aufgebracht sind, um die Dehnung der Stofflage zu überwachen. Beispielsweise wird in der DE 10 2008 042 554 A1 ein elastischer Brustgurt zur Überwachung der Atmung eines Patienten beschrieben, wobei auf den Brustgurt ein Dehnungssensor aufgenäht ist, der mit einer Auswerteeinheit auf dem Brustgurt verbunden ist.

Die JP 2014 025180 A offenbart eine Hose mit einem integrierten Dehnungssensor, der mithilfe eines elastischen Klebstoffes auf den Stoff der Hose aufgebracht ist. Über den Dehnungssensor können Dehnungen im Stoff der Hose registriert werden. Die Sensorsignale werden über einen ebenfalls in die Hose integrierten Sender auf ein externes Gerät übertragen.

Die CN 107 951 484 A zeigt eine mehrlagige Elektrode für ein Bekleidungsstück, welche eine elektrisch leitende Kontaktschicht und darunter liegend eine Leiterplatte aufweist, wobei zwischen der Kontaktschicht und der Leiterplatte eine isolierende Schaumstoffschicht angeordnet ist. Die Kontaktschicht ist über einen Leiter elektrisch mit der Leiterplatte verbunden, wobei der Leiter durch die Schaumstoffschicht hindurchgeführt ist.

Aus der WO 2022/061250 A2 ist ebenfalls eine mehrlagige Elektrode bekannt, die sich aus einer obenliegenden, elektrisch leitenden Schicht, einer darunterliegenden Filmschicht aus Polyethylen, einer Polsterschicht und einer untenliegenden Gummischicht zusammensetzt. Die elektrisch leitende Schicht kann als leitende Textilschicht oder leitendes Silikon ausgebildet sein.

Zum weiteren Stand der Technik wird auf die Druckschriften US 2020/0046246 A1 und DE 20 2014 104 995 U1 verwiesen.

Der Erfindung liegt die Aufgabe zugrunde, mit einfachen Maßnahmen eine sichere elektrische Verbindung zwischen einer Elektrode und einem elektrischen Leiter zu schaffen.

Diese Aufgabe wird erfindungsgemäß mit den Merkmalen der unabhängigen Ansprüche gelöst. Die abhängigen Ansprüche geben zeitmäßig Weiterbildungen an.

Die erfindungsgemäße Elektrode mit einem elektrischen Leiter kann beispielsweise für ein Bekleidungsstück, insbesondere für ein Unterbekleidungsstück oder gegebenenfalls auch für ein Oberbekleidungsstück eingesetzt werden, zum Beispiel für ein T-Shirt oder eine Sporthose. In Betracht kommt auch eine Verwendung für eine Bandage, insbesondere eine Gelenkbandage wie zum Beispiel eine Kniebandage oder eine Ellbogenbandage oder für einen am Körper zu tragenden Gurt. Eine Stofflage, mit der die Elektrode verbunden werden kann, kann Bestandteil des Bekleidungsstücks, des Gurts bzw. der Bandage sein, oder es wird die Stofflage mit dem Bekleidungsstück, dem Gurt oder der Bandage verbunden.

Die Elektrode besteht aus mehreren aufeinanderliegenden Lagen, die jeweils in parallelen Ebenen liegen. Eine Lage ist als eine elektrisch leitende Elektrodenschicht und eine weitere Lage ist als Schaumstoffschicht ausgebildet, die direkt oder indirekt mit der Elektrodenschicht verbunden ist. Zwischen die Elektrodenschicht und die Schaumstoffschicht ragt ein elektrischer Leiter hinein und steht in physischem und elektrischem Kontakt mit der Elektrodenschicht. Es können elektrische Impulse von der Elektrodenschicht über den elektrischen Leiter zu einer Auswerteeinheit übertragen werden, beispielsweise Vitalparameter wie Herzfrequenz oder elektrischer Hautwiderstand. Desweiteren ist es auch möglich, zur Stimulation elektrische Impulse über den elektrischen Leiter auf die Elektrodenschicht zu übertragen. Die Elektrodenschicht liegt beim Tragen des mit der Elektrode ausgestatteten Bekleidungsstücks, Gurts oder der Bandage unmittelbar auf der Haut des Trägers.

Die Ausführung der Elektrode mit der Schaumstoffschicht hat den Vorteil, dass der Tragekomfort verbessert ist. Die Schaumstoffschicht verleiht der Elektrode Stabilität und hält die Elektrodenschicht in Form, so dass außerdem ein flächiger Kontakt zwischen der Elektrodenschicht und der Haut des Trägers und damit einhergehend eine gute Impulsübertragung gewährleistet **ist.** Die Schaumstoffschicht ist gegebenenfalls in der Lage Feuchtigkeit aufzunehmen, so dass zum einen ein Feuchtigkeitsüberschuss von der Haut aufgesogen wird und zum andern der elektrische Kontakt mit dem Leiter verbessert wird, der zwischen der Schaumstoffschicht und der Elektrodenschicht **liegt.**

Die Elektrodenschicht ist als eine Textillage ausgeführt, die mit einem elektrisch leitenden Elastomer versehen **ist.** Das Elastomer ist beispielsweise als Silikon ausgeführt und kann elektrisch leitende Partikeln oder Fasern enthalten, beispielsweise aus Silber oder aus Karbon.

In einer vorteilhaften Ausführung ist der elektrische Leiter durch eine Ausnehmung in der Schaumstoffschicht hindurchgeführt, wobei ein Endabschnitt des elektrischen Leiters parallel zur Elektrodenschicht verläuft, so dass eine verhältnismäßig große Kontaktfläche zwischen Leiter und Elektrodenschicht gegeben ist. Das Hindurchführen des elektrischen Leiters durch die Ausnehmung in der Schaumstoffschicht hat den Vorteil, dass der elektrische Leiter nicht über den Seitenrand der Elektrodenschicht, sondern in platzsparender Weise von unten kommend über Schaumstoffschicht zugeführt wird. Alternativ sind auch Ausführungen möglich, in denen der elektrische Leiter über den Seitenrand der Elektrodenschicht zugeführt wird, was den Vorteil hat, dass der elektrische Leiter nicht umgebogen werden muss.

Der elektrische Leiter ist beispielsweise als ein flexibles, elektrisch leitendes Textilband ausgebildet, das einfach oder mehrfach umgebogen werden kann. Das elektrisch leitende Textilband ist zum Beispiel als Textilband mit elektrisch leitenden Fasern oder elektrisch leitenden Partikeln ausgebildet.

Zwischen der Elektrodenschicht und der Schaumstoffschicht befindet sich eine Textilschicht, auf der die Elektrodenschicht aufliegt. Ein Endabschnitt des elektrischen Leiters liegt zwischen der Textilschicht und der Elektrodenschicht und steht somit in Kontakt mit der Elektrodenschicht. In die Textilschicht kann eine Ausnehmung eingebracht sein, durch die der elektrische Leiter hindurchgeführt ist. Die Textilschicht weist vorzugsweise eine geringere Porosität als die Schaumstoffschicht auf. Feuchtigkeit wie beispielsweise Schweiß wird zunächst von der Textilschicht aufgenommen, bevor die Schaumstoffschicht erreicht wird, so dass die Schaumstoffschicht auch bei längerem Einsatz der Elektrode zumindest weitgehend trocken bleibt. Die Textilschicht kann elektrisch leitend oder elektrisch isolierend ausgebildet sein.

Gemäß noch einer weiteren vorteilhaften Ausführung ist mindestens eine Lage der Elektrode mit einer thermoadhäsiven Folie versehen, über die eine feste Verbindung mit einer benachbarten Lage hergestellt wird. Bei Erwärmung dringt Material der thermoadhäsiven Folie in die benachbarte Lage ein; nach der Aushärtung ist die gewünschte feste Verbindung zur benachbarten Lage hergestellt.

Es kann zweckmäßig sein, dass sowohl die Elektrodenschicht als auch die Schaumstoffschicht jeweils mit einer thermoadhäsiven Folie versehen sind. An der Elektrodenschicht befindet sich die thermoadhäsive Folie auf der der Schaumstoffschicht zugewandten Seite, um eine Verbindung mit der zugewandten Lage herzustellen, bei der es sich entweder um die Schaumstoffschicht, oder, in bevorzugter Ausführung um die handelt. Die Textilschicht kann ihrerseits ebenfalls eine thermoadhäsive Folie zur Verbindung mit der Schaumstoffschicht aufweisen. Die thermoadhäsive Folie an der Schaumstoffschicht befindet sich vorteilhafterweise auf der der Elektrodenschicht abgewandten Seite und dient zur Verbindung mit einer Stofflage des Bekleidungsstücks, der Bandage oder des Gurts.

In einer weiteren Ausführung weist die Schaumstoffschicht eine größere Dicke als die Elektrodenschicht auf. Dies hat den Vorteil, dass eine hohe Stabilität der Elektrode bei zugleich hohem Tragekomfort gewährleistet ist.

Die Dicke der Schaumstoffschicht kann in Abhängigkeit der jeweiligen Anforderung in einem weiten Bereich variiert werden. Die Dicke liegt beispielsweise bei 1 mm oder kleiner als 1 mm oder auch bei einem Wert bis 20 mm oder größer als 20 mm.

Die Schaumstoffschicht erstreckt sich über eine größere Fläche als die Elektrodenschicht. Die Schaumstoffschicht ragt über die Elektrodenschicht hinaus, wobei der hinausragende Randbereich sich beim Tragen unmittelbar auf der Haut abstützt, wodurch der Komfort verbessert werden kann.

Die Elektrodenschicht kann eine Länge von 100 mm oder größer als 100 mm aufweisen.

Als Material der Schaumstoffschicht können offenzellige Schaumstoffe, beispielsweise Polyurethanschaumstoffe oder Polyesterschaumstoffe verwendet werden. In Betracht kommen auch geschlossenzellige Schaumstoffe wie zum Beispiel Moosgummi, Neopolen, Polyethylen oder Zellgummi. Desweiteren sind gemischtzellige Schaumstoffe, insbesondere Hybridschaumstoffe aus offenzelligen und geschlossenzelligen Schaumstoffen möglich. Darüberhinaus kommen Integralschaumstoffe in Betracht, zum Beispiel Silikonschaumstoff, Polyetherschaumstoff, Kaltschaum/HR Schaumstoff (High Resistant) oder Latexschaum. Es können Schaumstoffe verwendet werden, die nur aus einem Material bestehen, oder Schaumstoffe aus mehreren Materialen, zum Beispiel mit Textil oder anderen Materialien kaschierte Schaumstoffe. Desweiteren können verschiedenartige Schaumstoffbeschaffenheiten eingesetzt werden wie zum Beispiel Noppenschaum.

Abhängig von den verwendeten Materialien für die verschiedenen Lagen kann die Elektrode ein elastisches Verhalten oder ein teilelastisches oder nichtelastisches Verhalten aufweisen, insbesondere in einer Richtung orthogonal zur Ebene der Lagen der Elektrode.

Für die verwendeten thermoadhäsiven Folien kommen Materialen wie beispielsweise Polyurethan, Polyamid, Polyester oder thermoplastische Elastomere in Betracht. Die Dicke der thermoadhäsiven Folien liegt beispielhaft in einem Wertebereich zwischen 10 Mikrometer bis über 500 Mikrometer.

Es kann desweiteren zweckmäßig sein, dass auf die Außenseite der Elektrode, insbesondere der Elektrodenschicht mit Elastomer, insbesondere Silikon ein Schriftzug oder Logo aufgebracht wird, vorzugsweise mithilfe einer Schablone oder gegebenenfalls mithilfe einer Düse. Das Elastomer kann gegebenenfalls zur besseren Lesbarkeit des Schriftzugs oder Logos einen Farbzusatz oder sichtbare Partikeln enthalten.

Weitere Vorteile und zweckmäßige Ausführungen sind den weiteren Ansprüchen, der Figurenbeschreibung und der Zeichnung zu entnehmen, die eine perspektivische Ansicht einer Elektrode zweigt, die aus mehreren Lagen Schichten aufgebaut ist und sich zur Verwendung auf einer Stofflage eignet, die beispielsweise Bestandteil eines Bekleidungsstücks, einer Bandage oder eines Gurts ist.

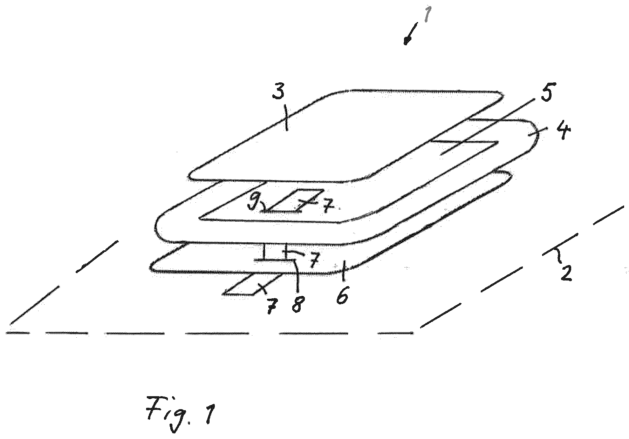

In Fig. 1 ist der schichtweise Aufbau einer Elektrode 1 mit mehreren parallelen Lagen dargestellt. Die Elektrode 1 kann als Sensor in einer Stofflage 2 verwendet werden, beispielsweise zur Erfassung von Vitalparametern oder zur Feststellung der Dehnung der Stofflage, aber auch zur Muskelstimulation. Die Stofflage 2 ist Teil eines Bekleidungsstücks, einer Bandage oder eines am Körper anzulegenden Gurts.

Die Elektrode 1 umfasst eine Elektrodenschicht 3, die als elektrisch leitfähige Textillage ausgeführt ist, insbesondere eine Textillage und ein elektrisch leitfähiges Elastomer, vorzugsweise Silikon umfasst. Die Elektrode 1 umfasst desweiteren eine Schaumstoffschicht 4 und eine zwischen der Elektrodenschicht 3 und der Schaumstoffschicht 4 liegende Textilschicht 5. Auf der Unterseite der Schaumstoffschicht 4 befindet sich eine thermoadhäsive Folie 6, die bei Erwärmung und anschließendem Abkühlen eine feste Verbindung zur Stofflage 2 eingeht, welche Träger der Elektrode 1 ist. Auch auf der Unterseite der Elektrodenschicht 3 kann sich eine thermoadhäsive Folie zur Verbindung mit der Textilschicht 5 befinden, ebenso auf der Unterseite der Textilschicht 5 zur Verbindung mit der Schaumstoffschicht 4.

Die Schaumstoffschicht 4 besitzt eine größere Dicke als die Elektrodenschicht 3. Die Schaumstoffschicht 4 weist außerdem eine größere Fläche auf als die Elektrodenschicht 3, so dass die Schaumstoffschicht 4 über die Elektrodenschicht 3 hinausragt und sich mit ihrem Randbereich zur Komfortverbesserung beim Tragen unmittelbar auf der Haut abstützen kann.

Zur elektrischen Kontaktierung der Elektrodenschicht 3 ist die Elektrode 1 mit einem elektrischen Leiter 7 versehen, der als elektrisch leitendes Textilband ausgebildet ist. Über den Leiter 7 können elektrische Impulse, die in der Elektrodenschicht 3 erzeugt werden, zu einer Auswerteeinheit übertragen werden. Umgekehrt ist es auch möglich, elektrische Impulse zur Haut- oder Muskelstimulation von einer Erzeugereinheit auf die Elektrodenschicht 3 zu übertragen.

Der bandförmige elektrische Leiter 7 ist durch Ausnehmungen 8, 9 geführt, die in Form eines Schlitzes in die thermoadhäsive Folie 6 und die Textilschicht 5 eingebracht sind. Auch in die thermoadhäsive Folie auf der Unterseite der Schaumstoffschicht 5 ist eine entsprechende, schlitzförmige Ausnehmung für den elektrischen Leiter 7 eingebracht. In die thermoadhäsive Folie auf der Unterseite der Elektrodenschicht 3 ist zweckmäßigerweise eine größere freie Fläche eingebracht, die mit dem an der Unterseite der Elektrodenschicht 3 anliegenden Endabschnitt des elektrischen Leiters 7 korrespondiert, so dass ein direkter Kontakt zwischen der Elektrodenschicht 3 und dem elektrischen Leiter 7 gewährleistet ist.

## Patentansprüche

1. Elektrode für ein Bekleidungsstück, einen Gurt oder eine Bandage, bestehend aus mehreren aufeinanderliegenden Lagen, wobei eine Lage als elektrisch leitende Elektrodenschicht (3) und eine weitere Lage als Schaumstoffschicht (4) ausgebildet ist, wobei die Elektrodenschicht (3) beim Tragen des mit der Elektrode ausgestatteten Bekleidungsstücks, Gurts oder der Bandage unmittelbar auf der Haut des Trägers liegt, wobei ein elektrischer Leiter (7) zwischen die Elektrodenschicht (3) und die Schaumstoffschicht (4) hineinragt und in Kontakt mit der Elektrodenschicht (3) steht,
wobei die Elektrodenschicht (3) eine Textillage und ein elektrisch leitendes Elastomer auf oder in der Textillage aufweist,
wobei sich zwischen der Elektrodenschicht (3) und der Schaumstoffschicht (4) eine Textilschicht (5) befindet, auf der die Elektrodenschicht (3) aufliegt und
wobei die Schaumstoffschicht (4) sich über eine größere Fläche erstreckt als die Elektrodenschicht (3).

2. Elektrode nach Anspruch 1,
**dadurch gekennzeichnet,**
**dass** der elektrische Leiter (7) durch eine Ausnehmung (9) in der Schaumstoffschicht (4) hindurchgeführt ist.

3. Elektrode nach Anspruch 1 oder 2,
**dadurch gekennzeichnet,**
**dass** der elektrische Leiter (7) durch eine Ausnehmung (9) in der Textilschicht (5) hindurchgeführt ist.

4. Elektrode nach einem der Ansprüche 1 bis 3,
**dadurch gekennzeichnet,**
**dass** der elektrische Leiter (7) als elektrisch leitendes Textilband ausgebildet ist.

5. Elektrode nach einem der Ansprüche 1 bis 4,
**dadurch gekennzeichnet,**
**dass** mindestens eine Lage (4) mit einer thermoadhäsiven Folie (6) zur Verbindung mit einer benachbarten Lage versehen ist.

6. Elektrode nach einem der Ansprüche 1 bis 5,
**dadurch gekennzeichnet,**
**dass** die Schaumstoffschicht (4) eine größere Dicke als die Elektrodenschicht (3) aufweist.

7. Stofflage-Elektrode-Kombination mit einer Elektrode (1) nach einem der Ansprüche 1 bis 6, wobei die Elektrode (1) mit einer Stofflage (2) eines Bekleidungsstücks, eines Gurts oder einer Bandage verbunden ist.

8. Bekleidungsstück, Gurt oder Bandage mit einer Stofflage-Elektrode-Kombination nach Anspruch 7.

## Claims

1. Electrode for a garment, belt or bandage, comprising several superimposed layers including one layer in the form of an electrically conductive electrode layer (3) and another layer in the form of a foam material layer (4), wherein during wearing of the garment, the belt or the bandage, provided with the electrode (1), the electrode layer (3) is in direct contact with the skin of a wearer, wherein an electrical conductor (7) extends between the electrode layer (3) and the foam material layer (4) and is in contact with the electrode layer (3), wherein the electrode layer (3) includes a fabric layer and an electrically conductive elastomer disposed on or in the fabric layer, wherein between the electrode layer (3) and the foam material layer (4) there is a fabric layer (5) on which the electrode layer (3) is supported and wherein the foam material layer (4) extends over a larger area than the electrode layer (3).

2. Electrode according to claim 1, **characterized in that** the electrical conductor (7) extends through an opening (9) in the foam material layer (4).

3. Electrode according to claim 1 or 2, **characterized in that** the electrical conductor (7) extends through an opening (9) in the fabric layer (5).

4. Electrode according to any of claims 1 to 3, **characterized in that** the electrical conductor (7) is in the form of an electrically conductive ribbon.

5. Electrode according to any of claims 1 to 4, **characterized in that** at least one layer (4) is provided with a thermo-adhesive foil (6) for a connection with an adjacent layer.

6. Electrode according to any of claims 1 to 5, **characterized in that** the foam material layer (4) is thicker than the electrode layer (3),

7. Fabric layer-electrode-combination with an electrode (1) according to any of claims 1 to 6, wherein the electrode (1) is connected to a fabric layer (2) of a garment, a belt or a bandage.

8. Garment, belt or bandage with a fabric layer-electrode-combination according to claim 7.

## Revendications

1. Électrode pour vêtement, ceinture ou bandage, composée de plusieurs couches superposées, dans laquelle une couche est conçue comme une couche d'électrode électriquement conductrice (3) et une autre couche comme une couche de mousse (4), dans laquelle la couche d'électrode (3) repose directement sur la peau du porteur lorsque le vêtement, la ceinture ou le bandage équipé de l'électrode est porté, dans laquelle un conducteur électrique (7) fait saillie entre la couche d'électrode (3) et la couche de mousse (4) et est en contact avec la couche d'électrode (3), dans laquelle la couche d'électrode (3) comporte une couche textile et un élastomère électriquement conducteur sur ou dans la couche textile, dans laquelle une couche textile (5) est située entre la couche d'électrode (3) et la couche de mousse (4), sur laquelle repose la couche d'électrode (3), et dans laquelle la couche de mousse (4) s'étend sur une surface plus grande que la couche d'électrode (3).

2. Électrode selon la revendication 1,
**caractérisée en ce que** le conducteur électrique (7) traverse une cavité (9) pratiquée dans la couche de mousse (4).

3. Électrode selon la revendication 1 ou 2,
**caractérisée en ce que** le conducteur électrique (7) traverse une cavité (9) pratiquée dans la couche textile (5).

4. Électrode selon l'une quelconque des revendications 1 à 3,
**caractérisée en ce que** le conducteur électrique (7) est conçu comme une bande textile conductrice.

5. Électrode selon l'une quelconque des revendications 1 à 4,
**caractérisée en ce qu'**au moins une couche (4) est munie d'un film thermoadhésif (6) permettant son collage à une couche adjacente.

6. Électrode selon l'une quelconque des revendications 1 à 5,
**caractérisée en ce que** la couche de mousse (4) présente une épaisseur supérieure à celle de la couche d'électrode (3).

7. Combinaison couche textile-électrode comprenant une électrode (1) selon l'une quelconque des revendications 1 à 6, l'électrode (1) étant reliée à une couche textile (2) d'un vêtement, d'une ceinture ou d'un bandage.

8. Un vêtement, une ceinture ou un bandage comprenant une combinaison couche de tissu-électrode selon la revendication 7.
